**Europäisches Patentamt**

**European Patent Office**

**Office européen des brevets**

(19)

(11) Veröffentlichungsnummer: **0 083 673**
**B1**

## (12) EUROPÄISCHE PATENTSCHRIFT

(45) Veröffentlichungstag der Patentschrift:
**24.06.87**

(21) Anmeldenummer: **82100130.2**

(22) Anmeldetag: **11.01.82**

(51) Int. Cl.⁴: **A 61 K 35/12,** A 61 K 39/00

(54) Erzeugnisse zur intravasalen Applikation von wasserlöslichen bzw. emulgierbaren antigenen Organextrakten.

(43) Veröffentlichungstag der Anmeldung:
**20.07.83 Patentblatt 83/29**

(45) Bekanntmachung des Hinweises auf die Patenterteilung:
**24.06.87 Patentblatt 87/26**

(84) Benannte Vertragsstaaten:
**AT BE CH DE FR GB IT LI LU NL SE**

(56) Entgegenhaltungen:
DE - A - 1 033 374
DE - A - 2 338 970
FR - A - 2 138 509
FR - M - 1 957

**CHEMICAL ABSTRACTS, Band 76, Nr. 21, 22. März 1972, Zusammenfassung 125294z, Seite 364, Columbus, Ohio, US, V.H. MORALES et al.: "Immunosuppresive action in vitro by a bovine spleen extract"**
**IVAN ROITT "Essential immunology", 4. Auflage, 1980, Blackwell Scientific Publications, Oxford, GB**
**CHEMICAL ABSTRACTS, Band 79, Nr. 7, 20. August 1973, Zusammenfassung 38638h, Seite 29, Columbus, Ohio, US, E. GARCIA-GIRALT: "Immunosuppressive effect of an inhibitor of DNA synthesis obtained from bovine spleen"**

(73) Patentinhaber: **Theurer, Karl Eugen, Prof.Dr.med.,**
**Brunnwiesenstrasse 23, D-7302 Ostfildern 1 (DE)**
Patentinhaber: **Theurer, Karl Georg, Dr.med.,**
**Brunnwiesenstrasse 23, D-7302 Ostfildern 1 (DE)**

(72) Erfinder: **Theurer, Karl Eugen, Prof.Dr.med.,**
**Brunnwiesenstrasse 23, D-7302 Ostfildern 1 (DE)**
Erfinder: **Theurer, Karl Georg, Dr.med.,**
**Brunnwiesenstrasse 23, D-7302 Ostfildern 1 (DE)**

## Beschreibung

Bei der tierexperimentellen und therapeutischen Anwendung von xenogenen, d.h. von artverschiedenen Individuen stammenden Totalextrakten aus verschiedenen Organarten wurde festgestellt, dass die therapeutische Wirkung dosis- und zeitabhängig ist. Insbesondere zeigt sich dies bei der Regression von Malignomen und Tumormetastasen. Es sind dazu wiederholte Behandlungen mit Dosierungen im mg- bis g-Bereich erforderlich. Diese Dosierung führt zur immunologischen Sensibilisierung, zu allergischen Reaktionen und zu einer Wirkungseinschränkung, insbesondere bei späteren Wiederholungsbehandlungen. Andererseits hat die Anwendung an Zellkulturen gezeigt, dass die Wirkung dort auch bei Wiederholungsbehandlungen reproduzierbar war und dass diese Wirkung durch Inhibition der Organextrakte mit den entsprechenden Antikörpern verhindert wird. Es war deshalb anzunehmen, dass auch in vivo die Blockierung der therapeutischen Wirkung der Organpräparate auf einer Antikörperbildung gegen dieselbe beruht. Die Erzeugung einer spezifischen Immuntoleranz gegen die therapeutisch wirksamen Organfaktoren war bei den relativ hohen erforderlichen Konzentrationen bisher nicht möglich.

Es wurde nun gefunden, dass durch intravasale Applikation von wasserlöslichen und emulgierbaren Organtrockenpulvern in ansteigender Dosierung und sich verlängernden Zeitabständen eine ausreichend stabile Toleranz für die wiederholte Anwendung der therapeutisch erforderlichen höheren Konzentration im mg- bis g-Bereich pro ml Lösungsmittel erreicht wurden. Solche Lösungen sind jedoch nicht haltbar und fallen rasch aus, so dass sie unmittelbar vor der Anwendung aus voll löslichen bzw. emulgierbaren Organtrockenpulvern hergestellt werden müssen.

Erfindungsgemäss werden nun pharmazeutische Erzeugnisse bereitgestellt, die eine oder mehrere verdünnte wässerige Lösungen dieser Organsubstanzen im Konzentrationsbereich pg bis µg/ml Injektionslösung und die entsprechenden Trockensubstanzen mit Lösungsmittel, die zur Herstellung von Injektionslösungen im Konzentrationsbereich von mg bis g/ml Injektionslösung, enthalten.

Die immunologische Toleranz bedeutet im Gegensatz zum Begriff der Arzneimittel-Toleranz (vgl. A. SCHREY: Toleranz bei Nitratpräparaten: Med. Klinik A.25.S.24/699 (1981) nicht eine Verringerung der pharmakologischen Wirksamkeit der angewandten Präparate, vielmehr wird gerade diese durch die immunologische Toleranz gegen das Arzneimittel erhalten. Es erscheint möglich, dass an der Entstehung von Arzneimittel-Toleranz im pharmakologischen Sinne ebenfalls immunologische Reaktionen gegen das Arzneimittel beteiligt sind. Diese werden durch Erzeugung einer immunologischen Toleranz, d.h. die Unterdrückung der Antikörperbildung gegen das Arzneimittel verhindert.

Die Toleranzerzeugung gegen xenogene Antikörperseren, wie sie aus der Serum-Therapie von Infektionskrankheiten bekannt ist, betrifft vollkommen andere Indikationen und keine Inhaltsstoffe von Zellgeweben. Im Prinzip sollen dadurch allergisch-anaphylaktische Reaktionen verhindert werden. Sie dient also nicht der Erhaltung der therapeutischen Wirksamkeit der angewandten Antikörperseren (vgl. W. GRONEMEYER, H. SCHMIDT in K. HANSEN: Lehrbuch der Allergie, Georg Thieme Verlag Stuttgart, 1957).

Auch die Desensibilisierung mit sogenannten Organ-Dilutionen im Rahmen der Zytoplasmatischen Therapie (vgl. Leitfaden der Zytoplasmatischen Therapie: vitOrgan Arzneimittel GmbH, 7302 Ostfildern 1 b. Stuttgart) besitzt unterschiedliche Indikationen und Anwendungsformen. Es handelt sich dabei um keine echten Lösungen und Emulsionen der Organsubstanzen, vielmehr enthalten diese noch korpuskuläre Bestandteile. Deshalb können die Konzentrationen im ng- und µg-Bereich nicht i.v. angewandt werden. Desgleichen auch nicht die Suspensionen aus den Trockensubstanzen. Suspensionen von diesen können nur intramuskulär appliziert werden. Die mitverwendeten korpuskulären Zellbestandteile wirken als Adjuvans der gelösten Faktoren immunogen und verhindern eine Toleranzerzeugung gegen das Präparat. Auch werden zusätzlich immunologische Adjuvantien zur Verstärkung der Antikörperbildung mitverwendet, um besonders bei degenerativen Erkrankungen und geriatrischen Indikationen einen organspezifischen Reiz durch die entstehenden Antikörper zu erzeugen. Bei der erfindungsgemässen Anwendung der vollöslichen und emulgierbaren Organtrockenpulver wird jedoch diese Antikörperbildung vermieden. Deshalb unterscheiden sich auch die Indikationen. Der Zustand der induzierten immunologischen Toleranz bleibt solange erhalten, als das Antigen in ausreichender Konzentration im Organismus vorhanden ist. Deshalb muss zur Erzeugung einer stabilen immunologischen Toleranz und deren Aufrechterhaltung das Antigen in mg- bis g-Konzentrationen fortlaufend injiziert werden. Bei Kombinationsbehandlungen mit Antigenmischungen aus verschiedenen Organen müssen deshalb die Organfaktoren gegen die die Toleranz erwünscht ist, fortlaufend verabfolgt werden, während man andere Faktoren gegen die eine Toleranz nicht mehr benötigt wird, nicht weiter anzuwenden braucht. Nach Aussetzen der tolerogenen Applikation von mehr als 5 Tagen ist mit einem Toleranzverfall zu rechnen. Eine Wiederholungsbehandlung macht es deshalb notwendig, erneut die Toleranz durch einschleichende Dosierungen wieder aufzubauen. Zur Prüfung der Toleranz bzw. einer möglichen Sensibilisierung gegen die verwendeten Präparate können diese als Antigen für Antigen-Antikörper-Reaktionen in vivo oder in vitro benutzt werden. Bei vorliegender Sensibilisierung muss die Behandlung mit umso höheren Verdünnungen wieder begonnen werden, je ausgeprägter die Sensibilisierung ist. Umso kürzer müssen dann auch die Applikationsabstände gewählt werden.

Normalerweise wird die Konzentration über pg, ng, μg auf mg und eventuell g/ml Organlösung gesteigert, nachdem man vor der i.v.-Applikation einen Sensibilitätstest durch konjunktivale Anwendung oder i.c.-Injektion durchführt. Bei positivem Testergebnis sollte eine Desensibilisierung bei i.c.-, s.c.- oder oraler Applikation nach den Gesichtspunkten der Allergologie durchgeführt werden. Bei reaktionsfreier Verträglichkeit der Konzentration μg kann dann die Applikation der ansteigenden Konzentrationen, beginnend von pg, i.v. erfolgen. Es ist möglich, Faktoren gegen die eine Antikörperbildung unterbunden werden soll zunächst tolerogen zu dosieren und nach eingetretener Toleranz dann andersartige Faktoren, die indirekt über Antikörper zur Wirkung gelangen, wie z.B. Faktoren die durch das Verfahren zur Isolierung von wirksamen Faktoren aus biologischen Geweben (EP-A-29 893) gewonnen wurden, immunogen zu dosieren. Dabei bleibt dann die vorher induzierte Toleranz gegen die zuerst angewandten andersartigen Faktoren erhalten, sodass sich die Antikörperbildung ausschliesslich gegen die immunogen dosierten Faktoren richtet.

Die Zelltherapie nach NIEHANS unterscheidet sich von vorliegender Anwendung durch die Verwendung von Zellsuspensionen, während hier voll lösliche und emulgierbare Organpräparate verwendet werden. Zellpräparate können nicht i.v. appliziert werden.

Die wiederholte Injektionsimplantation grösserer Mengen kann zur pathogenen Allergisierung oder aber zur Immunparalyse führen. Letztere bedeutet eine Blockierung des Immunsystems, sodass auch erwünschte Antikörper ausbleiben. Wiederholungsbehandlungen sind erst nach Monaten möglich, wenn sich die Sensibilisierung spontan verloren hat.

Die voll wasserlöslichen Organtrockenpulver enthalten kristallisierte Proteide, Proteine, Nukleinsäuren (Ribonukleinsäuren und Desoxyribonukleinsäuren), Peptide, Enzyme, Polysaccharide und Lipide, sowie deren Untereinheiten und Bestandteile. Es hat sich gezeigt, dass Mischungen von nativen Makromolekülen mit ihren Untereinheiten die Erzeugung von Toleranz gegen die angewandten Präparate erleichtern. Ebenso wirken sich chemische Veränderungen wie z.B. schonende Hydrolyse und Sulfatierung, Phosphorylierung, Halogenisierung, Nitrierung, Carboxylierung, Äthylierung, Alkylsubstitution, aromatische Substitution, Alkylierung, Arylierung, günstig aus und verbessern die Wirksamkeit gegenüber nativen wässrigen Frischhomogenaten oder -lyophylisaten um bis zu 60%. Die Wasserstoffionenkonzentration und die Temperatur während der Aufarbeitung sind für die Wirksamkeit des Endproduktes ebenfalls entscheidend (vgl. EP-A-29 893). Wie erwähnt können auch isolierte Teilfaktoren aus einer Organart, wie auch Organkombinationen angewandt werden. Es ist auch möglich, das Molekulargewicht durch Filtration einzustellen und die Präparate z.B. nach dem Eiweissgehalt oder anderen Wirkfaktoren zu standardisieren. Optimale Wirkungen wurden mit voll wasserlöslichen Organtrockenpulvern erzielt, die durch Lyophilisation eingestellter Lösungen erhalten wurden, die durch hochtourige Zentrifugation und Filtration aus Suspensionen von sulfatierten Trockenpulvern (EP-A-29 893) gewonnen werden. In analoger Weise können aber auch Suspensionen von lyophilisierten Trockenpulvern wie auch Frischhomogenate aus Organgeweben aufgearbeitet werden. Das Verhältnis von Proteinen bzw. Proteiden zu Polysacchariden, Nukleinsäuren und Lipiden untereinander kann festgelegt werden. Durch Mitverwendung von Emulgatoren bzw. Detergentien, z.B. Natriumdodecylsulfat in höheren Verdünnungen, die nicht denaturieren, werden Lipide emulgierbar. Auch ist eine partielle Extraktion der Organvollextrakte durch Lipidlösungsmittel wie auch ein enzymatischer Abbau unerwünschter Faktoren möglich.

Prinzipiell sind alle intravasalen Applikationsformen zur Anwendung geeignet. Grundvoraussetzung ist die Induktion und Erhaltung von immunologischer Toleranz über eine sogenannte Low-Zone-Toleranz (vgl. J.H. HUMPHREY und R. WHITE: Kurzes Lehrbuch der Immunologie: Georg Thieme Verlag, Stuttgart), die jedoch bis zu höheren Konzentrationen im mg- bis g-Bereich erweitert wird. Die alleinige Low-Zone-Toleranz wird sonst aufgrund von Verdünnungen von pg bis μg durch die therapeutisch erforderlichen Konzentrationen der Organextrakte durchbrochen. Ebenso wichtig sind auch die sich verlängernden Applikationsabstände von Stunden bis höchstens 3–5 Tagen bei gleichzeitig ansteigender Dosierung und die i.v.-Injektion bzw. -Infusion. Dieses Vorgehen besitzt gegenüber der Erzeugung von High-Zone-Toleranz den Vorteil, dass die erwünschten Reaktionen des Immunsystems gegen andere Antigene, z.B. Tumor- oder Infektions-spezifische Antigene nicht beeinträchtigt werden, also keine allgemeine Immunparalyse eintritt. Trotzdem erfolgt durch ansteigende Dosierung auf Konzentrationen von mg bis g/ml Lösungsmittel eine Erweiterung der Toleranz auf phylogenetisch verwandte Antigene. Es entsteht dabei eine Art Überkreuz-Toleranz bezüglich der xenogenen Antigene und allogener bzw. individueller Antigenqualitäten wie sie in den körpereigenen, entsprechenden Organarten vorkommen. Dies eröffnet weitere wichtige Indikationsgebiete dieser immunologisch tolerogenen Anwendung von voll wasserlöslichen bzw. emulgierbaren Organtrockenpulvern aus unterschiedlichen Organarten, einzeln und in Mischungen.

Die Indikationen der beanspruchten Erzeugnisse sind krankheitsbedingte oder durch Vorbehandlung mit Zelltherapie entstandene immunopathogenen Autosensibilisierungen und Autoaggressionen. Es erfolgt dort eine Desensibilisierung und Toleranzerzeugung mit analogen Organarten, gegen die eine Antikörperbildung besteht. Die wiederholte systemische Applikation grösserer Organmengen im mg-Bereich führt gegenüber der bisher nur einmaligen Anwendung der höheren Konzentrationen zur Verbesserung der therapeutischen Wirkung und zur Ausheilung.

Genetisch oder chromosomal bedingte Erkrankungen bedürfen einer ständigen Substitution von Faktoren aus Stoffwechsel-gesunden Zellen und Geweben in einer Dosierung die sonst die Gefahr einer Antikörperbildung gegen die wirksamen Faktoren einschliesst. Bei der Zelltherapie nach NIEHANS sind deshalb Wiederholungsbehandlungen beim Down-Syndrom nur in Abständen von frühestens ½ Jahr bis 1 Jahr möglich. Die immunologisch tolerogene Anwendung erlaubt nun eine Dauerbehandlung.

Bei Organtransplantaten kann durch vorherige Erzeugung und fortlaufende Unterhaltung einer stabilen Überkreuz-Toleranz gegen allogene und individuelle Organ-Antigene die bisher bestehende Immunbarriere durchbrochen werden. Die Aufrechterhaltung der gegen xenogene oder allogene Organfaktoren induzierten Toleranz macht die Organtransplantation zeitlich unabhängig. Es muss aber mindestens 5 Tage vor der Organverpflanzung eine intensive Vorbehandlung in Form der Schnellerzeugung von Toleranz durch sehr kurze Applikationsintervalle der Anstieg zur Volldosierung durchgeführt werden. Bei längerem Abstand bis zur Organtransplantation muss die Toleranz durch weiter ansteigende Dosierung verstärkt und aufrecht erhalten werden. Ebenso auch nach erfolgter Organtransplantation. Die erreichte Organtoleranz kann durch Verwendung der applizierten Organpräparate oder von Blut- bzw. Gewebezellen des Spenders als Antigen vor und nach der Organtransplantation durch Antigen-Antikörper-Reaktion überprüft werden.

Bei Organinfarkten, z.B. vom Herzmuskel und parenchymatösen Organen sowie Apoplexien bieten sich ebenfalls vollkommen neue Möglichkeiten durch die präventive, möglichst frühzeitige Toleranzerzeugung in der Zeit bis zum nekrotischen Zerfall des infarzierten Gewebes. Die Freisetzung der Zellinhaltsstoffe die zur Autosensibilisierung führt, erfolgt erst nach einigen Tagen. Die entstehenden Autoantikörper werden für Komplikationen des Heilungsverlaufs und für das Auftreten von Reinfarkten verantwortlich gemacht. Die möglichst frühzeitige Induktion von Überkreuz-Toleranz verhindert die Entstehung von Autoantikörpern und damit diese Komplikationen. Die tolerogene Behandlung muss deshalb gleich nach dem Infarktereignis einsetzen und bis zur Abheilung durchgeführt werden. Einer Kombinationsbehandlung mit anderen intensiv-therapeutischen Methoden steht nichts im Wege.

Zur Prävention von Operationsfolgen, die ebenfalls auf der Basis einer Autosensibilisierung beruhen, kann ähnlich wie bei der Organtransplantation mit Präparaten analog denen, die bei dem chirurgischen Eingriff geschädigt werden, mindestens 5 Tage vor Operation eine Überkreuz-Toleranz erzeugt werden.

Bei Verbrennungen steht kein längeres Intervall bis zur Bildung von Autoantikörpern zur Verfügung. Trotzdem kann auch hier gleich eine entsprechende Behandlung mit Präparaten analog den verbrannten Geweben stattfinden. Bei Verbrennungen wie auch bei Operationsfolgen geht es aber auch darum, die Intensität der Sensibilisierungsvorgänge durch Organpräparate, insbesondere aus foetalem Thymus, foetaler Milz, foetalem Anteil der Plazenta, Nebenniere und Lymphknoten in Form von Einzel- oder Kombinationspräparaten zu verringern. Die tolerogene Dosierung ist hier auf die eingesetzten Präparate bezogen und nicht auf die eigentlich geschädigten Organe. Diese allgemeine biologische Immunsuppression kommt bei allen Erkrankungen in Betracht, die auf Autoantikörperbildung beruhen, insbesondere auch bei Erkrankungen der Gelenke und des Zentralnervensystems. Sie kann als Kombinationsbehandlung zusammen mit der gezielten Toleranzerzeugung gegen bestimmte Organe oder Organkombinationen durchgeführt werden.

Es erscheint möglich, dass es im Gegensatz zu Adjuvantien, die die Immunogenität verstärken, auch solche gibt, die die Toleranzbildung verstärken können. Zu denken ist dabei an Nukleotide oder Nukleoside oder permeabilitätsverändernde Stoffe wie z.B. Dinatriumchromoglycat. Solche toleranzbegünstigende Adjuvantien können bei den angegebenen Indikationen zusätzlich mitverwendet werden. Für die Behandlung für Krebskrankheiten erscheint jedoch eine allgemeine Immunsuppression oder die zusätzliche Anwendung solcher toleranzbegünstigender Medikamente nicht geeignet, weil dadurch die phylakogene Immunabwehr beeinträchtigt werden. Die folgenden Beispiele erläutern die praktische Anwendung.

Beispiel 1

Die Behandlung von malignen Tumoren (z.B. des Mammakarzinoms mit Knochenmetastasen) erfolgt mit Einzel- oder Kombinationspräparaten aus maternem Anteil der Plazenta, jugendlichem Thymus, Leber, Milz, Pankreas, Gehirnanteilen, Nabelstrang oder Epiphyse zusammen mit dem Organ des Primärtumors oder dem Sitz der Metastasen, in diesem Falle Brustdrüse und Knochenmark, zusammen mit jugendlichem Testes ohne Spermatogenese wegen der Hormonabhängigkeit des Tumors oder mit Mischungen aus mindestens 2 der eingangs genannten Komponenten zu gleichen Teilen bei immunologisch tolerogener Dosierung der wässrigen Lösungen in Verdünnungen pg, ng, µg und mg aus den voll wasserlöslichen und emulgierbaren Organtrockensubstanzen bzw. -pulvern. Durch langsame i.v.-Injektion oder Tropfinfusion mit üblichen Infusionslösungen, denen die Präparate zugesetzt sind, werden am 1. Tag der Behandlung 4–6 ml, am 2. Tag 6 ml der Verdünnung pg, am 3. und 4. Tag je 6 ml der Konzentration ng, am 5. Tag 6 ml der Konzentration µg und am 8. Tag 2 ml, am 11., 15. und 19. Tag je 4 ml und danach 2mal wöchentlich (Montag und Donnerstag) je 2 ml der Konzentration mg/ml, ansteigend bis zu einer Gesamtmenge pro Applikation von 1 g appliziert. Bei stationärer Behandlung kann die Anwendung intensiviert werden. Am 1. Tag 10–20 ml der Konzentration pg verteilt auf 2–3 Gaben, am 2. Tag 10–20 ml der Konzentration ng, verteilt auf 2 Gaben und am 3. Tag 10–20 ml der Konzentration µg, sodann am 4. Tag 2–4 ml, am 7.

Tag 4–10 ml, am 9. Tag 10–20 ml der Konzentration mg/ml. Danach dann 2mal wöchentlich (Montag, Donnerstag) je 2–6 ml der Konzentration mg, gegebenenfalls ansteigend auf eine tägliche Gesamtmenge von 1 g. Zusätzlich werden Präparate der gleichen Organzusammensetzung in Konzentration mg/ml in Kapseln oder in Liposomen, Suspensionen, oral 3mal täglich 8–10 Tropfen verabreicht.

Die Behandlung kann als Intervallbehandlung bis zum Verschwinden der Symptome oder als 3-Wochen-Kur durchgeführt werden. Eine Wiederholung ist bei erneuter Verschlechterung des Krankheitszustandes jederzeit möglich, sonst aber in sich verlängernden Abständen von 2, 3, 4 und 6 Monaten. Zur Tumorprävention bei Risikopatienten werden Wiederholungskuren in Abständen von ½ bis 1 Jahr durchgeführt.

Vor der ersten i.v.-Applikation empfiehlt es sich auch bei Wiederholungsbehandlungen eine Vortestung der Reaktionslage durch i.c.-/s.c.-Injektionen von 2 ml der Konzentration pg vorzunehmen. Bei ausbleibender Reaktion kann dann 1–4 Stunden danach mit der i.v.-Applikation begonnen werden. Bei der Daueranwendung kann nach der 3-wöchigen Behandlung auf i.m. oder s.c.-Injektionen eventuell mit Retard- oder Depot-Präparaten übergegangen werden. Diese sollen pro Tag Wirkstoffmengen von µg bis g freisetzen.

Bei Androgen-hormonabhängigen Prostatakarzinom werden neben Prostata-Präparaten, die dem Mutterboden der Metastasen entsprechenden und solche aus Ovar verwendet. In ähnlicher Weise verfährt man auch bei anderen Tumorarten.

In Tierversuchen an Mäusen, Ratten und Hamstern wurde festgestellt, dass die tolerogen dosierten löslichen Organextrakte zu einer signifikanten Verbesserung der therapeutischen Wirkung bezüglich der Regression der Tumormenge, dem Ausbleiben von Metastasen und der Allgemeinwirkung führen gegenüber der Anwendung von Organpräparaten mit korpuskulären, nicht löslichen Bestandteilen. Nach anfänglicher Tumorregression beginnt dort 4–6 Wochen nach Behandlungsbeginn ein erneutes Tumorwachstum. Dieses wird durch die tolerogene Anwendung von voll löslichen Organextrakten verhindert.

Beispiel 2

Erzeugung von Toleranz durch Erzeugnisse mit voll wasserlöslichen Organextrakten, die unspezifisch eine überschiessende immunologische Reaktionslage mit der Tendenz zur verstärkten Antikörperbildung normalisieren und die bei exogenen Allergien wie auch bei immunopathogenen Autoimmunerkrankungen, bei Verbrennungen, Organtransplantaten und gegen Operationsfolgen angewandt werden, ohne dass eine spezifische Toleranz gegen die pathogenen Antigene oder Allergene erzeugt wird. Es werden dazu voll lösliche Organtrockensubstanzen, bzw. -pulver aus foetalem Thymus, foetaler Milz, foetalem Anteil der Plazenta, Nebenniere und Lymphknoten, einzeln oder in beliebiger Mischung aa verwendet.

Diese Präparate können auch gleichzeitig mit der spezifischen Desensibilisierung und Toleranzerzeugung gegen exogene wie endogene pathogene Antigene oder aber mit der sogenannten Gegensensibilisierung unter Verwendung von zum Antigen umgewandelten Antikörpern bzw. Reaginen angewandt werden.

Das Behandlungsschema unterscheidet sich vom Beispiel 1 durch die Anwendung von jeweils nur 2–4 ml der verschiedenen Verdünnungsstufen. Die Behandlung wird nach dem Verschwinden der Symptome 1–2 Wochen weitergeführt.

Die Wirkung dieser Therapie konnte am Absinken der Immunglobuline E und dem Ansteigen der Reaktionsschwelle für die Auslösung allergischer Reaktionen festgestellt werden. Es ist wahrscheinlich, dass der Therapieerfolg auch bei allergisch disponierten Patienten dauerhafter ist, als bei der reinen Desensibilisierung und der Verwendung von nicht wasserlöslichen Organpräparaten, bei nicht tolerogener Dosierung.

Beispiel 3

Zur Desensibilisierung und Erzeugung von Überkreuz-Toleranz bei immunopathogenen Autoaggressionskrankheiten wie z.B. der chronischen Nephritis oder den Kollagenosen werden voll lösliche Präparate analog den Organarten verwendet, gegen die sich die Autoantikörper richten. Die Behandlung erfolgt zunächst s.c. oder i.m. mit ansteigender Dosierung von pg über ng zu µg. Bei starker Symptomatik am 1. Tag evtl. 3 und am 2. Tag 2mal täglich in Abständen von 2–3 Stunden, sodann einmal täglich jeweils 1–4 ml. Bei guter Verträglichkeit wird zur nächst höheren Konzentration übergegangen. Nach Erreichen der µg-Konzentration wird die i.v.-Applikation beginnend mit der höchsten Ausgangsverdünnung von jeweils 2–4 ml der ansteigenden Konzentration bis mg/ml als Injektion oder als Infusion eingesetzt. Eine gleichzeitig kombinierte Anwendung von Präparaten entsprechend Beispiel 2 beschleunigt den Therapieerfolg. Dieser konnte objektiviert werden durch Absinken der bestehenden Eosinophilie, der Lymphozytose und der Autoantikörper.

Beispiel 4

Zur Erzeugung von Überkreuz-Toleranz vor einer allogenen Nierentransplantation wird mindestens 5 Tage vor der Transplantation, möglichst aber 2–3 Wochen vorher, mit Präparaten aus foetaler und jugendlicher Schweineniere in ähnlicher Weise wie in Beispiel 1 behandelt. Es können dabei Mischpräparate entsprechend Beispiel 2 zusätzlich mitverwendet werden. Zur Aufrechterhaltung einer stabilen Toleranz werden alle 3–4 Tage bis zu g-Mengen in Konzentrationen mg/ml zunächst i.v. appliziert.

Tierexperimentell konnte bei der Überpflanzung von Rattenhaut auf Mäuse eine signifikante Verlängerung der Überlebenszeit der Transplantate durch die Vorbehandlung der Mäuse mit wasserlöslichen Extrakten aus Rattenhaut, gegenüber nichtbehandelten Tieren nachgewiesen werden.

Beispiel 5

Zur Erzeugung von Überkreuz-Toleranz bei Organinfarkten und Apoplexien werden Präparate analog der betroffenen Organart möglichst frühzeitig nach dem Infarktereignis bei rascher Konzentrationssteigerung wie in Beispiel 1 appliziert. Gleichzeitig können Mischpräparate entsprechend Beispiel 2 angewandt werden. Die Behandlung wird bis zur klinischen Ausheilung und Normalisierung der Laborparameter fortgesetzt. Bei Apoplexien erfolgt ab dem 3. Tag zusätzlich die intrathekale Anwendung von jeweils 1–2 ml der Verdünnungen pg und ng. Die Behandlung kann zusätzlich zu der üblichen Intensivtherapie durchgeführt werden. Die Konzentrationen und Applikationsmengen dürfen nur bei einwandfreier Verträglichkeit gesteigert werden, sonst muss die Konzentration und die Applikationsmenge mindestens um 1–2 Stufen, d.h. Dezimalen gesenkt werden. Dies gilt für alle Indikationen.

**Patentansprüche**

1. Pharmazeutische Erzeugnisse zur intravasalen Applikation von wasserlöslichen bzw. emulgierbaren antigenen Organextrakten aus Einzelorganen oder Organkombinationen mit nativen molekularen und haptenen Bestandteilen bzw. Untereinheiten fetalen und/oder juvenilen, xenogenen oder allogenen Ursprungs im Konzentrationsbereich von mg bis g/ml Injektionslösung, dadurch gekennzeichnet, dass sie eine oder mehrere verdünnte wässerige Lösungen dieser Organsubstanzen im Konzentrationsbereich pg bis µg/ml Injektionslösung und die entsprechenden Trockensubstanzen mit Lösungsmittel, die zur Herstellung von Injektionslösungen im Konzentrationsbereich von mg bis g/ml Injektionslösung erforderlich sind, enthalten.

2. Erzeugnisse nach Anspruch 1, gekennzeichnet durch die Mitverwendung von immunbiologischen Adjuvantien.

3. Erzeugnisse nach Anspruch 1–2 mit verbesserter Löslichkeit und verringerter Immunogenität durch Sulfatierung, Phosphorylierung, Halogenisierung, Nitrierung, Carboxylierung, Äthylierung, Hydrierung oder Substitution von aromatischen oder alkylierenden Substanzen.

4. Erzeugnisse nach Anspruch 1–3, gekennzeichnet durch die zusätzliche Verwendung von Extrakten aus fetalem Thymus, Nebenniere, fetaler Milz, fetalem Anteil der Plazenta (Chorion), Trophoblast, fetalen Lymphknoten, einzeln oder als Mischung.

**Claims**

1. Pharmaceutical products for intravascular administration of water-soluble or emulsible antigenic organ extracts from individual organs or combinations of organs with native molecular and haptene components or subunits of fetal and/or juvenile, xenogenic or allogenic origin in the concentration range from mg to g/ml injection solution, characterized in that they contain one or more dilute aqueous solutions of these organ substances in the concentration range from pg to ug/ml injection solution and the corresponding dry substances with solvents, which are required to produce the injection solutions in the concentration range from mg to g/ml injection solution.

2. Products according to claim 1 characterized by the additional inclusion of immunobiological adjuvants.

3. Products according to claims 1 and 2 with improved solubility and reduced immunogenicity as a result of sulfation, phosphorylation, halogenation, nitration, carboxylation, ethylation, hydration or substitution of aromatic or alkylating substances.

4. Products according to claims 1, 2, 3 characterized by the additional use of extracts of fetal thymus, adrenals, fetal spleen, fetal portion of the placenta (chorion), trophoblast, fetal lymphnodes, individually or as a mixture.

**Revendications**

1. Produits pharmaceutiques pour l'application intravasale d'extraits d'organe antigènes hydrosolubles ou émulsifiables provenant d'organes individuels ou d'associations d'organes, comportant des constituants ou sous-unités natifs moléculaires et haptènes d'origine fétale et/ou juvénile, xénogène ou allogène dans des concentrations allant de mg à g/ml de solution injectable, caractérisés par le fait qu'ils comportent une ou plusieurs solutions aqueuses diluées de ces substances d'organe en concentrations allant de pg à µg/ml de solution injectable et les substances sèches correspondantes avec leur solvant nécessaire pour la préparation de solutions injectables dans les concentrations allant de mg à g/ml de solution d'injection.

2. Produits selon revendication 1, caractérisés par l'emploi simultané d'adjuvants immuno-biologiques.

3. Produits selon revendications 1 à 2 présentant une solubilité améliorée et une immunogénéité abaissée par sulfatage, phosphorylation, halogénisation, nitrification, carboxylation, éthylation, hydrogénation ou substitution de substances aromatiques ou alkylantes.

4. Produits selon revendications 1 à 3, caractérisés par l'emploi complémentaire d'extraits provenant de thymus fétal, de surrénales, de rate fétale, de la portion fétale du placenta (chorion) de trophoblastes, de ganglions lymphatiques fétaux, utilisés individuellement ou sous forme de mélanges.